(19) Europäisches Patentamt
European Patent Office
Office européen des brevets

(11) Publication number: **0 435 265 A2**

(12) # EUROPEAN PATENT APPLICATION

(21) Application number: **90125533.1**

(22) Date of filing: **27.12.90**

(51) Int. Cl.5: **C08G 63/08**, C08G 59/34, C07C 69/675, C07D 303/16, C07C 265/04, C09D 167/04, C09D 163/00, C08G 18/68, C08G 18/42, C08F 299/04

(30) Priority: **27.12.89 US 457922**

(43) Date of publication of application: **03.07.91 Bulletin 91/27**

(84) Designated Contracting States: **DE FR GB IT SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **Koleske, Joseph Victor**
**1513 Brentwood Road**
**Charleston, West Virginia 253149(US)**
Inventor: **Armstrong, George Harvey**
**153 Kellyridge Road**
**New Kensington, Pennsylvania 15068(US)**
Inventor: **Ashcraft, Arnold Clifton, Jr.**
**R.D. No. 1, Box 355**
**Hightstown, New Jersey 08520(US)**

(74) Representative: **Behrens, Dieter, Dr.-Ing. et al**
**Wuesthoff & Wuesthoff Patent- und Rechtsanwälte Schweigerstrasse 2**
**W-8000 München 90(DE)**

(54) **Unsaturated polylactones and derivatives thereof.**

(57) Novel unsaturated polylactones are produced by the reaction of an unsaturated alcohol, such as tetrahydrobenzyl alcohol, with a lactone. Said product may be subsequently epoxidized to form unique polylactone epoxides. Also, the unsaturated polylactones may be derivatized with isocyanates, anhydrides, and carboxylic acids and epoxidized to form useful modified polylactone epoxides. The novel compounds of this invention are useful in coating, ink, adhesive, or sealant formulations, as well as useful as intermediates in the production of other valuable compounds, and in other end uses.

EP 0 435 265 A2

# UNSATURATED POLYLACTONES AND DERIVATIVES THEREOF

## BACKGROUND OF THE INVENTION

Unsaturated alcohols corresponding to the structural formula,

(I)

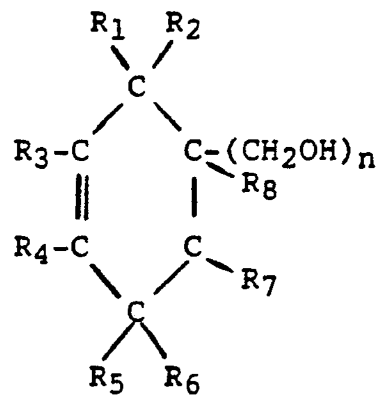

wherein $R_1$ through $R_8$ are the same or different and are hydrogen, phenyl, or unsubstituted or substituted alkyl or cycloalkyl groups of one to about 8 carbon atoms, and n has a value of one or two, are known. Such compounds are disclosed, for example, in H. Lee and K. Neville, page 3-16, "Handbook of Epoxy Resins," McGraw-Hill Book Co., NY (1967) and U. S. Pat. No. 3,833,665 (1974) issued to A. J. Papa and W. R. Proops.

Also known to those skilled in the art is the reaction of lactones, such as epsilon-caprolaotone, with active hydrogen initiators to produce the corresponding derivatives, such as the caprolactone polyols and the caprolactone acrylates. Such compounds are described in U.S. Pat. 3,169,945 and 4,683,287. However, not previously suggested nor disclosed are the polylactone derivatives and epoxidized polylactone derivatives of Formula I compounds nor their derivatives nor their range of useful properties.

## SUMMARY OF THE INVENTION

The novel polylactone derivatives of this invention are those defined by the following structural formulas. The unsaturated polylactone compound is described by structural Formula II:

(II)

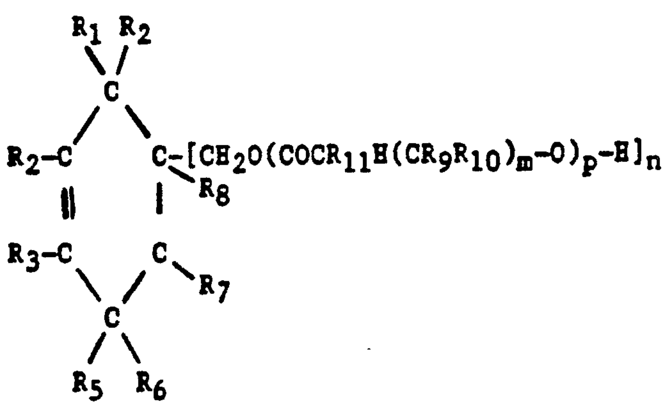

where $R_9$, $R_{10}$, and $R_{11}$ are the same or different and are hydrogen, phenyl, or lower alkyl having 1 to about 5 carbon atoms with the proviso that not more than three of the $R_9$, $R_{10}$, and $R_{11}$ groups are alkyl or phenyl groups, and m is an integer of from zero to about 12, n has a value of 1 or 2, and the average value of p, as will be described in detail below, has a value of from zero to about 30 or more, preferably from 1 to about 10. It is preferred that $R_3$ and $R_4$ be hydrogen.

The novel polylactone epoxide derivatives of this invention are those defined by structural Formula III:

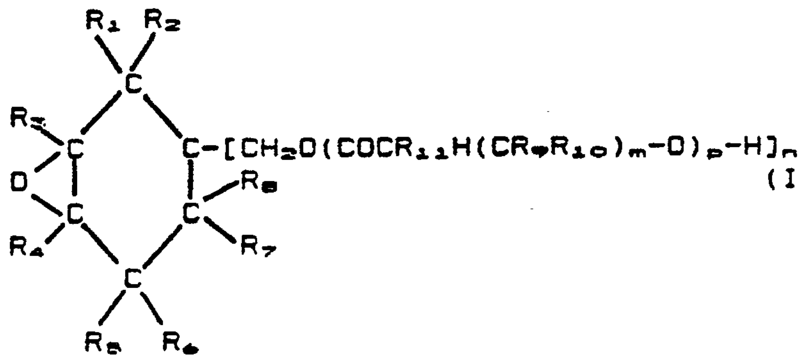

(III)

where $R_1$ through $R_{11}$, m, p, and n are as described above.

Other novel, useful derivatives of the unsaturated polylactone compounds and of the polylactone epoxides of this invention are those defined by the structural Formulas IV and V:

$$(F)_w(-OC\overset{H}{\overset{|}{N}})_x-(B)_z \qquad (IV)$$

where F is a Formula II compound without the active hydrogen atom of the terminal hydroxyl group, B is the residue of a monofunctional or polyfunctional isocyanate molecule when its isocyanate groups are removed, x has a value of 1 to 6, preferably from 1 to 3, and z has a value of 1 or 2, preferably a value of 1, and w has a value of 1 to 3, and

$$(F-\overset{O}{\overset{\|}{C}}-)_Y-A \qquad (V)$$

where F is as described above, A is the residue of a polyfunctional carboxylic acid without its carboxyl groups, or of an anhydride without the anhydride group (-CO-O-CO-), and Y has a value of 1 to 3. For example, if the polycarboxylic acid were adipic acid, A would be-$CH_2CH_2CH_2CH_2$-. For these derivatives, it is understood by those skilled in such art that when n = 2 a variety of products can be obtained and it is preferred that n = 1.

Novel, useful multifunctional polylactone epoxide derivatives of this invention are those defined by structural Formula VI and VII,

$$(E)_w(-OC\overset{H}{\overset{|}{N}})_x-(B)_z \qquad (VI)$$

$$(E-\overset{O}{\overset{\|}{C}}-)_y-A \quad , \qquad (VII)$$

where E is an epoxidized form of F and is denoted by the structural Formula VIII,

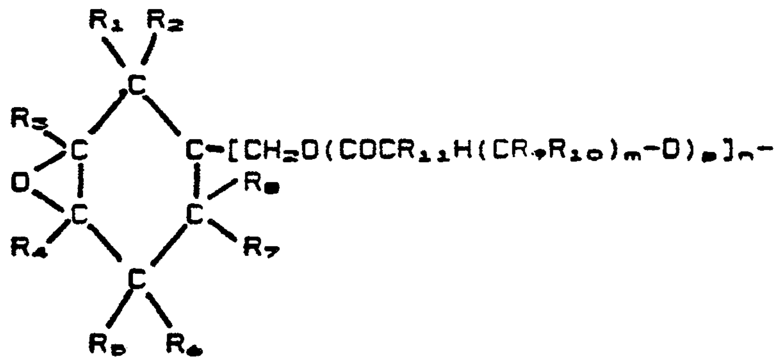

(VIII)

When homopolymerized or copolymerized, the above described compounds are useful in a variety of ways, including as components in coatings, adhesives, inks, and sealants. Such products are used in various industrial products such as electrodepositable automobile primers, automobile topcoats, can coatings, furniture coatings, decorative coatings, inks for pictorial or written communication, electrical or electronic coatings, sealants, and the like. The coatings containing these compounds can be applied by conventional techniques, such as spray or roll coating and can be cured by a variety of techniques, including actinic radiation, exposure to thermal energy, and the like.

The polycaprolactone epoxides of this invention can also be used as carboxylic and other acid scavengers in their prepared form or by anchoring them through the hydroxyl group to a polymer, or to any of a variety of suitable substrates reactive with a hydroxyl group.

## DESCRIPTION OF THE INVENTION

### The Unsaturated Polylactones (II)

The novel unsaturated polylactones (II), polylactone epoxides (III), and derivatives thereof (IV, V, VI, and VII) of this invention are preferably produced by the catalytic reaction of an unsaturated alcohol (I) with a lactone or mixture of lactones at an elevated temperature, as more fully described below.

The unsaturated alcohols (I) suitable for use in production of the unsaturated polylactone and polylactone epoxides of this invention include tetrahydrobenzyl alcohol (also known as 3-cyclohexene-1-methanol),

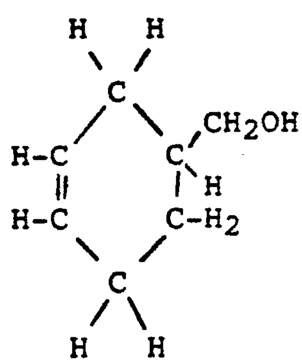

(IX)

,

3-cyclohexene-1, 1-dimethanol,

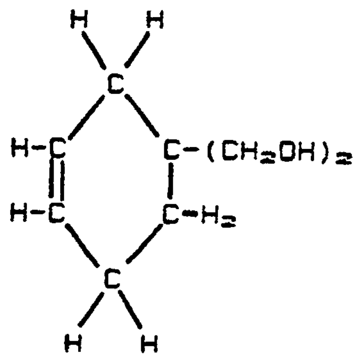

(X)

6-methyl-3-cyclohexene-1-methanol,

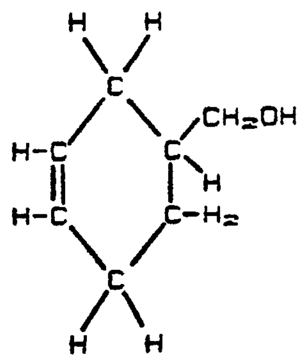

(XI)

,

6-methyl-3-cyclohexene-1,1-dimethanol, 4,6-dimethyl-3-cyclo-hexene-1-methanol, 2-butyl-3-cyclohexene-1,1-dimethanol, 2,6-dimethyl-2-phenyl-3-cyclohexene-1-methanol,and the like.

The lactones suitable for use in the production of the unsaturated polylactone, polylactone epoxides, and derivatives thereof are cyclic ester compounds of the general structure

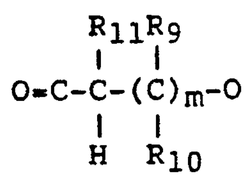

(XII)

where $R_9$, $R_{10}$, and $R_{11}$ are hydrogen, phenyl, or lower alkyl of one to about five carbon atoms with the proviso that not more than three of these R groups are phenyl or lower alkyl and preferably that not more than two of these R groups are phenyl or lower alkyl, and most preferably that all these R groups are hydrogen; and m is an integer of from zero to about 12 and preferably from three to five, and most preferably four. Illustrative of the lactones that can be used are epsilon-caprolactone, 3,5-dimethyl-epsilon-caprolactone, 6-methyl-epsilon-caprolactone, 3-isopropyl-epsilon-caprolactone, 5-phenyl-epsilon-caprolactone, 6-phenyl-2-methyl-epsilon caprolactone, delta-valerolactone, 5- methyl-delta-valerolactone, beta-propriolactone, zeta- enantholactone, eta-caprylolactone, and the like, as well as mixtures of these lactones. The most preferred lactone is epsilon-caprolactone.

The unsaturated polylactone compounds of Formula II are prepared by reacting the lactone (XII) with the unsaturated alcohol (I) in the presence of about 500 parts per million, preferably less than 200 parts per million, most preferably less than about 50 parts per million, based on the combined weight of lactone and unsaturated alcohol, of a catalyst. The catalyst that may be used herein includes Lewis acids and protonic acids such as one or more organometallic compounds and other metallic compounds. Illustrative of the catalysts one can mention stannous octanoate, dibutyltin dilaurate, stannous chloride, boron trifluoride, boron trifluoride etherate, hexafluorophosphoric acid, triflic acid, triflic acid salts such as diethylammonium triflate, stannic chloride, ferric chloride, zinc octanoate and other zinc salts, tetraisopropyl titanate, butyl titanate, and the like. Preferred catalysts include stannous octanoate, dibutyltin dilaurate, zinc octanoate, tetraisopropyl titanate, butyl titanate, and the like.

The reaction is carried out at a temperature of from about 100 °C to about 200 °C, preferably from about 110 °C to 170 °C, and most preferably from 120 °C to 160 °C. The reaction may be carried out at atmospheric pressure, although higher or lower pressures may be used. The reaction is carried out for a period of from about 2 to about 24 hours, preferably from about 3 to 12 hours.

Although the unsaturated alcohol, lactone, and catalyst may be combined at room temperature and heated to reaction temperature, it is preferred that the process be carried out by adding the lactone to a reaction vessel and heating this to reaction temperature, about 110 °C to 170 °C, while sparging the lactone with an inert gas such as nitrogen to remove water or other low molecular weight impurities. The lactone may be dried with, for example, conventional agents such as molecular sieves before adding it to the reaction vessel. At the end of the reaction period, the unsaturated polylactone product (II) may be collected and stored for future use or it may be epoxidized as described below. It is understood by those skilled in the art that the value of p in structural Formula II is a variable that has various values in any given product that is described by an average value of p. That is, a distribution of molecular weights, whose characteristics depend on the ratio of lactone to unsaturated alcohol used, the reaction temperature and the reaction time, will be obtained. Illustratively, a product that has an average value of p equal to two may contain species which have values for p that range from zero to about ten or more.

<u>The Isocyanate Modified Unsaturated Polylactones</u>

Derivatives that can be made from the unsaturated polylactone compounds (II) include urethane-linked compounds (IV and VI). The catalyzed or uncatalyzed reaction of the unsaturated polylactone compounds with multifunctional isocyanates produces a multi-unsaturated derivative that contains urethane groups (IV). The addition of such linkages in a compound can provide altered polarity, improved adhesion, improved abrasion resistance, improved dyeability, and other useful attributes including increased functionality and higher reactivity or enhanced cure rates in products prepared from the compound or derivatives prepared from the urethane-modified compound. This can be illustrated by the following equation, in which OCN-B-NCO represents a diisocyanate, that illustrates the reaction of 2 moles of II and one mole of a diisocyanate:

$$2\ \mathrm{II} + \mathrm{OCN{-}B{-}NCO} \longrightarrow \mathrm{F{-}\overset{\overset{O}{\|}}{C}{-}\overset{\overset{H}{|}}{N}{-}B{-}\overset{\overset{H}{|}}{N}{-}\overset{\overset{O}{\|}}{C}{-}F} \qquad (\mathrm{XIII})$$

If desired, monoisocyanates can be used to react with and cap all or a portion of the hydroxyl functionality of II. This is illustrated below when the monoisocyanate B-NCO is used and q, which can have a value equal to or less than n, is equal to n.

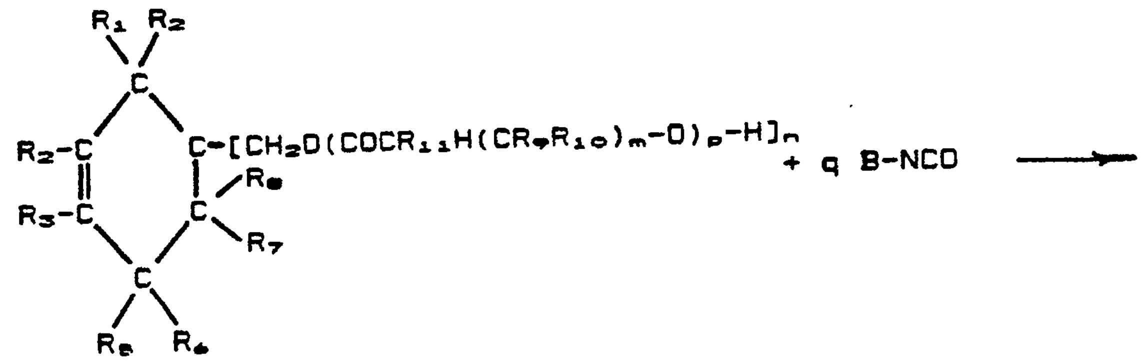

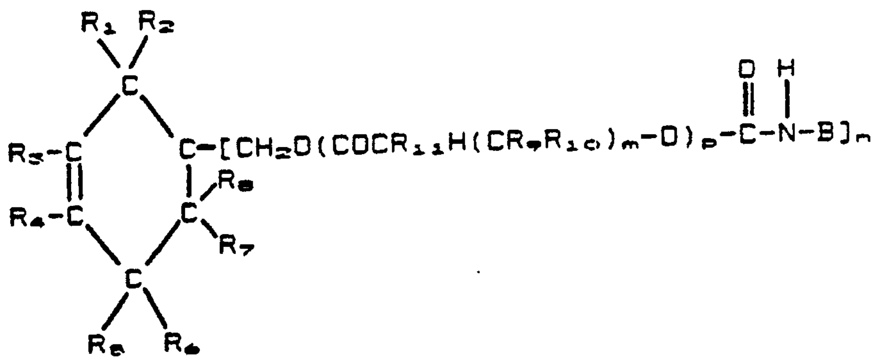

(XIV)

The polyisocyanates that can be used in this invention are well known to those skilled in the art and should not require detailed description herein, but illustrative thereof one can mention 2,4-toluene diisocyanate and 2,6-toluene diisocyanate (TDI), 4,4'- diphenylmethane diisocyanate (MDI), 4,4'-dicyclohexylmethane diisocyanate or reduced MDI, meta- and para-tetramethyl xylene diisocyanate, 3-isocyanatomethyl-3,5,5-trimethylcyclohexyl-isocyanate, hexamethylene diisocyanate, 2,2,4- and 2,4,4-trimethylenehexamethylene-disocyanate, 4,4',4"-triisocyanato triphenylmethane, and the like. Any of the polyisocyanates can be used alone or in admixture with other isocyanates, including monoisocyanates. Illustrative of monoisocyanates one can mention methyl isocyanate, ethyl isocyanate, chloroethyl isocyanate, chloropropyl isocyanate, chlorohexyl isocyanate, norbornene isocyanate, hexyl isocyanate, phenyl isocyanate, butyl isocyanate, isopropyl isocyanate, octadecyl isocyanate, naphthyl isocyanate, vinyl isocyanates such as the isopropenyl-alpha,alpha-dimethylbenzyl isocyanates, and the like.

The amount of unsaturated polylactone (II) used can be an amount sufficient to allow reaction of the isocyanato groups with up to 1.0 equivalent of the total number of hydroxyl groups present. Thus, from 0.025 to 1.0 isocyanato equivalent is reacted per hydroxyl equivalent, preferably from 0.2 to 1.0 isocyanato equivalent per hydroxyl equivalent, and most preferably from 0.5 to 1.0 isocyanato equivalent per hydroxyl equivalent initially charged. In certain special instances when multifunctional isocyanates are employed, isocyanato functionality in excess of 1.0 isocyanato equivalent per hydroxyl equivalent may be used so that residual free isocyanato functionality remains and the novel compounds may be used in moisture-curing urethane systems. The conventional urethane reaction catalysts are used to increase reaction rate if desired.

The reaction of the unsaturated polylactones (II) with isocyanate is conducted at a temperature of from about 25° C to about 120° C, preferably from about 40° C to about 70° C. The time of reaction will vary depending on the particular reactants charged, lack or use of added catalyst, temperature, and the batch size of the reaction mixture, facts which are well known to those skilled in the art. Generally, a reaction period of from about 1 to 12 or more hours at from about 40° C to 80° C is adequate to produce the isocyanate-modified products (IV and XIV).

<u>The Anhydride or Carboxylic Acid Modified Unsaturated Polylactones</u>

The catalyzed or uncatalyzed reaction of the unsaturated polylactone compounds (II) with a polycarboxylic acid anhydride or a multifunctional carboxylic acid produces a multi-unsaturated derivative that contains ester groups and the residue of the anhydride or carboxylic acid compound (V and VII). The addition of such linkages into a compound can provide altered polarity, dyeability, water resistance, flexibility, and

other useful attributes as well as increased functionality and thereby improved reactivity and cure rates in products prepared from the compound or derivatives prepared from the modified compound. This can be illustrated by the following equation, which describes preparation of a polycarboxylic acid anhydride modified unsaturated polylactone (XV).

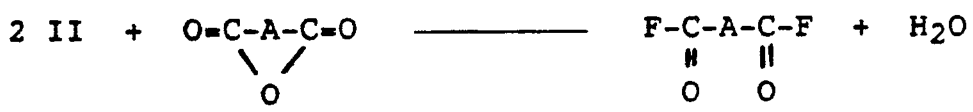

(XV)

If a dicarboxylic acid is used to link the unsaturated polylactone molecules (II), the following equation depicts the resultant reaction and ester-linked product (XVI).

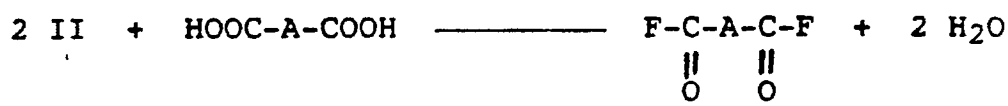

(XVI)

If desired, monocarboxylic acids or acetic anhydride can be used to react with and cap all or a portion of the hydroxyl functionality of II. This is illustrated below when the monocarboxylic acid A-COOH is used and q, which can have a value equal to or less than n, is equal to n.

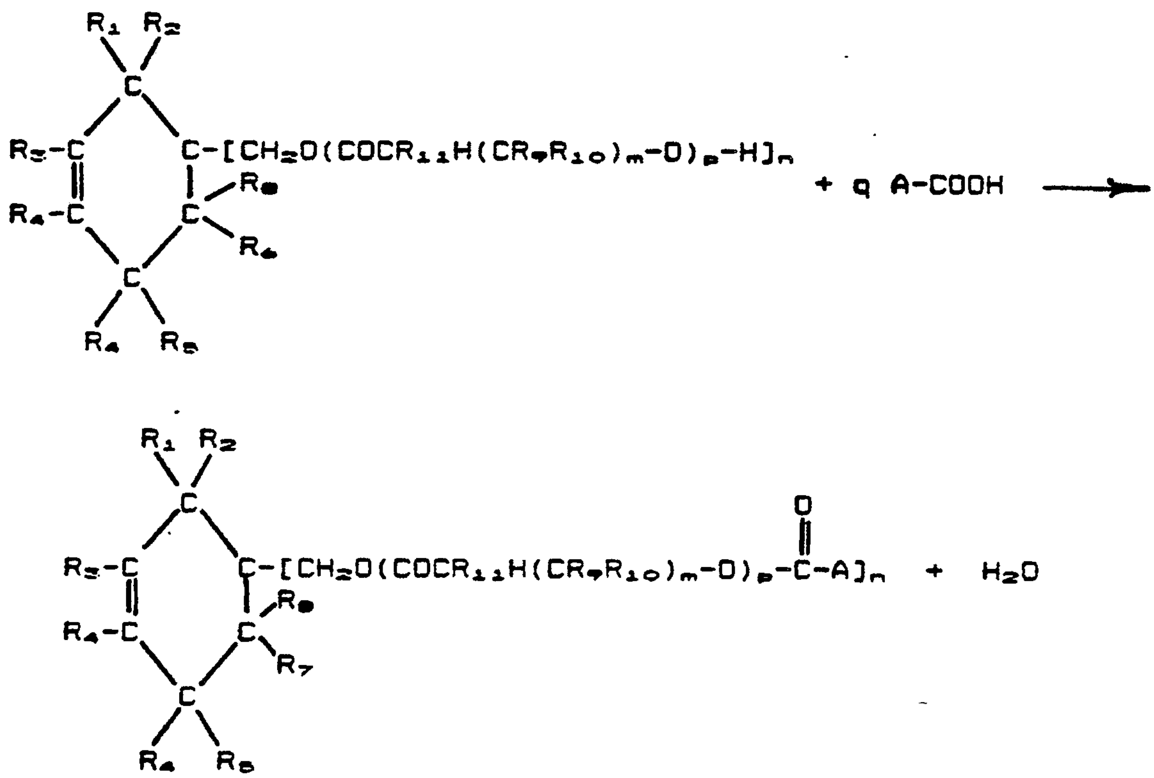

(XVII)

If acetic anhydride had been used to cap the molecule, acetic acid instead of water would have been

formed and the group A would be a methyl group.

Illustrative of suitable polycarboxylic acid anhydrides that can be used, one can mention trimellitic anhydride, tetrahydrophthalic anhydride, phthalic anhydride, isophthalic anhydride, benzophenone dicarboxylic acid anhydride, maleic anhydride, succinic anhydride, glutaric anhydride, napthoic anhydride, chlorendic anhydride, acetic anhydride, or any other intramolecular anhydride, including those having substituents thereon such as halogen atoms, alkyl or alkoxy groups, nitro, carboxyl, aryl or any other group that will not unduly interfere with the reaction.

Illustrative of the polycarboxylic and monocarboxylic acids that can be used are maleic acid, phthalic acid, isophthalic acid, hexahydrophthalic acid, terahydrophthalic acid, methyl-tetrahydrophthalic acid, trimellitic acid, acetic acid, propionic acid, butyric acid, hexanoic acid, lauric acid, tridecanoic acid, behenic acid, benzoic acid, palmitic acid, oleic acid, cinnamic acid, acrylic acid, methacrylic acid, linoleic acid, stearic acid, and the like, as well as mixtures of such acids.

The amount of polycarboxylic acid anhydride, polycarboxylic acid, acetic anhydride, or monocarboxylic acid or mixture of these components that is reacted with the unsaturated polylactone II can be an amount sufficient to permit reaction with all of the hydroxyl groups present in a reaction mixture; however, it is most preferred to use an amount which is insufficient to react with all of the hydroxy groups present in the reaction mixture to ensure a low acid number in the final product. Low or nil acid numbers will ensure good stability of the polylactone epoxides derivatives prepared from these compounds. The amount used can vary from about 0.1 to 1.0 anhydride or carboxylic acid equivalent per hydroxyl equivalent, preferably from about 0.3 to 1.0 anhydride or carboxylic acid equivalent per hydroxyl equivalent, and most preferably from about 0.7 to about 1.0 anhydride or carboxylic acid equivalent per hydroxyl equivalent present in the reaction mixture. If desired, mixtures of anhydrides and carboxylic acids can be used. In the reaction, conventional esterification catalysts that are well known to those skilled in the art can be used.

The unsaturated polylactone is reacted with the polycarboxylic acid anhydride, polycarboxylic acid, acetic anhydride, or monocarboxylic acid at a temperature of from about 60 °C to 220 °C, preferably from about 90 °C to 170 °C. The time required for reaction will vary depending upon the particular reactants charged, the temperature, and the batch size of the reaction mixture, facts which are well known to those skilled in the art of condensation reactions. The reaction is usually conducted until an acid number of less than about 0.1 is obtained.

## The Polylactone Epoxides

The novel polylactone epoxides (III, VI, VII) of this invention are prepared by reaction of the unsaturated polylactones (II, IV, V, XIII, XIV, XV, XVI, XVII) of the invention with an epoxidizing agent such as peracetic acid. The methods of oxidation and formation of epoxides is readily accomplished by those skilled in the art. Methods of epoxidation are described in U. S. Pat. Nos. 2,716,123, 2,745,847, and 2,750,395, in C. A. May and Y. Tanaka, "Epoxy Resins Chemistry and Technology," Marcel Dekker, Inc., NY (1973), and in H. Lee and K. Neville, "Handbook of Epoxy Resins," McGraw-Hill Book Co., NY (1967).

Epoxidizing agents of various types can be used. These agents can be formed in situ from hydrogen peroxide and an organic acid such as acetic acid or can be preformed and used as a peracid. Illustrative of the peracids that can be used in carrying out epoxidations are perbenzoic acid, peracetic acid, perpropionic acid, perbutyric acid, percaproic acid, perlactic acid, permonochloroacetic acid, permonosuccinic acid, t-butylperbenzoic acid, and the like. When used, the peracids are usually dissolved in a solvent such as ethyl acetate to minimize explosive and other hazards.

The unsaturated lactone compound is reacted with the epoxidizing agent at temperatures of less than about 5 °C to about 90 °C, preferably at temperatures of about less than about 25 °C to 60 °C. The time required for reaction will vary depending upon the particular reactants charged, the temperature, and the batch size of the reaction mixture, facts which are well known to those skilled in the art of epoxidation chemistry. In general, the peracid solution is carefully and very slowly added to the reactor containing the unsaturated polylactone, in either a neat form or dissolved in a suitable solvent such as ethyl acetate, which is held at a constant reaction temperature. Rate of peracid addition should be such that a desired maximum temperature is not exceeded. The exothermic oxidation reaction that takes place is controlled by cooling the reactants to the desired reaction temperature. Peracid addition rate is decreased or stopped if necessary to maintain temperature control. A method of quenching the reaction is usually made available and maintained as, for example, in the laboratory an ice/water bath is available. The reaction product is then isolated by vacuum stripping of the acetic acid that is formed and the solvent that had been used to dissolve the unsaturated polylactone and/or peracid. If desired, the product may be redissolved and reisolated by vacuum stripping using conventional techniques.

The unsaturated polylactones of the invention can be coupled by reaction with isocyanates, anhydrides or carboxylic acids and then epoxidized as described above or in the case of hydroxyl-terminated, unsaturated polylactones, the compounds can be epoxidized to form compounds of Formula III and then coupled with diisocyanates. In the case of isocyanate, anhydride, or carboxylic acid modified derivatives, the preferred process is to form first the derivatives and then to epoxidize the unsaturated compounds.

Formulated Compositions Using Polylactone Epoxides

The polylactone epoxides (III, VI, and VII) and the unsaturated polylactones (II, IV, V, XIII, XIV, XV, XVI, XVII) can be formulated to produce coating, ink, and sealant compositions by the addition of other reactants, polyols, crosslinking agents, pigments, fillers, surfactants, slip agents, and other additives conventionally used in the production of compositions. The polylactone epoxides can cure into coating, adhesive, ink, and sealant compositions by reaction of the epoxide group with suitable crosslinking agents such as carboxylic acids of various types, polyols, etc., and by reaction of the terminal hydroxyl group with suitable crosslinking functionality such as epoxide, isocyanate, aminoplast, etc. The unsaturated polylactones can cure or react into coatings, adhesives, inks, and sealants by reaction of the terminal hydroxyl group with suitable crosslinking functionality such as epoxide, isocyanate, aminoplast, etc. The unsaturation in these molecules can also be used for crosslinking purposes, or for reaction with other compounds such as halogens, which would impart flame retardance characteristics, and so on. Although this invention is not meant to be limited by any particular mechanism, the above mechanism, as well as other mechanisms, are offered as realistic descriptions of how various reactions occur as the products are made and used.

Although homopolymers can be made from the polylactone epoxides, usually they will be used in combination with other compounds and the final product formed through a copolymerization process. Formulations will contain from about 1 to about 100 weight percent polylactone epoxide, preferably from about 1 to about 40 weight percent polylactone epoxide. Other formulations will contain from about 1 to about 60 weight percent of the unsaturated polylactones, preferably from about 1 to about 40 weight percent of the unsaturated lactones. Still other formulations will contain mixtures of the unsaturated polylactones and the polylactone epoxides. For example, the polylactone epoxides can be used alone, can be combined with each other, and/or can be combined with other epoxides, polyols, and vinyl ethers to form useful coating films after copolymerization has taken place. The compositions can be used or formulated for a broad variety of end uses including automotive coatings, can coatings, general metal coatings, decorative coatings, electronics coatings including solder masks, photoresists, and conformal coatings, protective coatings for compact and optical discs, and the like, as well as inks for printed words or designations, for pictorial representation, and the like, for molded objects such as printing plates, and for sealants used in the automotive, home and electronics industries.

Coatings can be applied by various techniques, illustrative of which are spray coating, roll coating of various types, dip coating, electrodeposition, brush, and the like. Particularly useful application methods are spray coating, dip coating, and electrodeposition. The coatings can be cured by a variety of techniques including radiation, thermal, air drying, and the like, depending on the particular system being formulated.

In producing coatings curable with actinic radiation and preferably ultraviolet light, the polylactone epoxides are combined with other cycloaliphatic epoxides, Novolac epoxides, and the like; vinyl ethers; polyols; onium salt, diazonium salt or other cationic photoinitiators; and, if needed, surfactants and other compounds known to those skilled in the art of coating formulation. The formulated coatings may contain inert solvents for the purpose of decreasing viscosity and improving application characteristics, or inert polymers, fumed silicas, and the like, to thicken the formulated coating and make it useful in screen printing or other operations. The coatings are cured by exposure to ultraviolet light radiation from a medium pressure mercury vapor lamp with the radiation between about 220 and 400 nanometers.

Suitable cycloaliphatic epoxides for combination with the polylactone epoxides of this invention are those having an average of one or more epoxide groups per molecule. Preferably the cycloaliphatic epoxide will be a mixture of epoxides that will usually contain a major proportion of cycloaliphatic epoxides with two or more epoxy groups per molecule. These cycloaliphatic epoxides will be present in from about 0 to about 95 weight percent of the formulation, preferably from about 30 to about 90 weight percent of the formulation, and most preferably from about 40 to 85 weight percent of the formulation. Illustrative of suitable cycloaliphatic epoxides are the following.

A 3,4-epoxycyclohexylmethyl-3,4-epoxycyclohexane carboxylate having the formula:

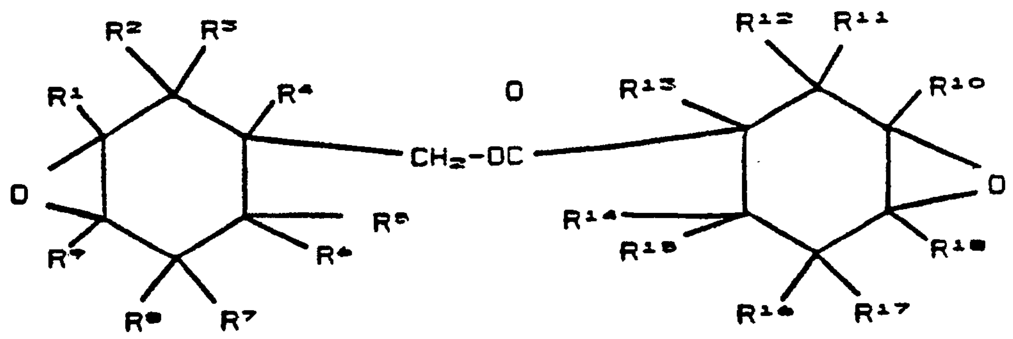

(XVIII)

wherein $R^1$ through $R^{18}$, which can be the same or different, are hydrogen or alkyl radicals generally containing one to six carbon atoms inclusive, and preferably containing one to three carbon atoms inclusive, as for example methyl, ethyl, n-propyl, i-propyl, n-butyl, s-butyl, i-butyl, t-butyl, hexyls, and the like. Particularly desirable compounds are those wherein $R^1$ through $R^{18}$ are hydrogen. Among specific compounds falling within the scope of Formula XVIII are the following: 3,4-epoxy-cyclohexylmethyl-3,4-epoxycyclohexane carboxylate; 3,4-epoxy-1-methylcyclohexylmethyl-3,4-epoxy-1-methylcyclohexane carboxylate; 6-methyl-3,4- epoxycyclohexylmethyl-6-methyl-3,4-epoxycyclohexane carboxylate; 3,4-epoxy-3-methylcyclohexylmethyl-3,4-epoxy-3-methylcyclohexane carboxylate: 3,4-epoxy-5-methylcyclohexylmethyl-3,4-epoxy-5-methyl-cyclohexane carboxylate. Other suitable compounds are described in, for example, U.S. Patent No. 2,890,194.

Diepoxides of cycloaliphatic esters of dicarboxylic acids having the formula:

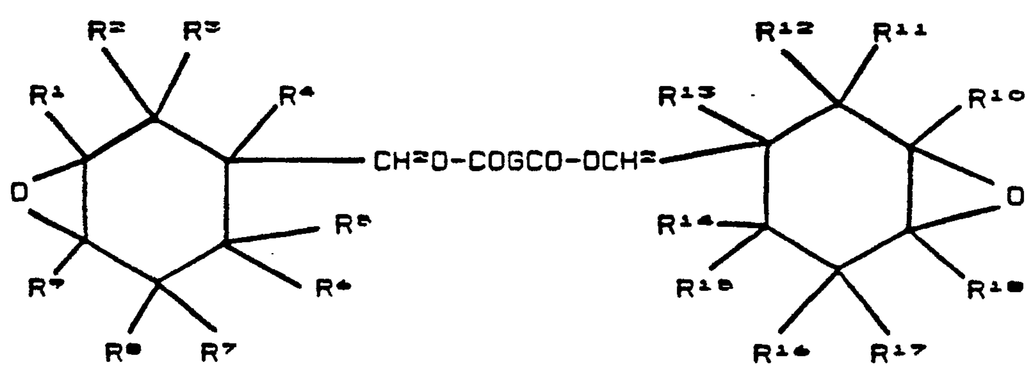

(XIX)

where $R^1$ through $R^{18}$ which can be the same or different are as defined for $R^1$ through $R^{18}$ in Formula XVIII above; G is a valence bond or a divalent hydrocarbon radical generally containing one to 10 carbon atoms, inclusive, and preferably, containing three to six carbon atoms, inclusive, as for example alkylene radicals, such as trimethylene, methyltrimethylene, tetramethylene, pentamethylene, hexamethylene, 2-ethylhexamethylene, octamethylene, nonamethylene, and the like; cycloaliphatic radicals such as 1,4-cyclohexane, 1,3-cyclohexane, 1,2-cyclohexane, and the like. Particularly desireable epoxides, falling within the scope of Formula XIX, are those wherein $R^1$ through $R^{18}$ are all hydrogen and G is alkylene containing three to six carbon atoms. Among specific diepoxides of cycloaliphatic esters of dicarboxylic acid are bis(3,4-epoxycyclohexylmethyl)oxylate, bis(3,4-epoxycyclohexylmethyl)adipate, bis(3,4-epoxy-6-methylcyclohexylmethyl)adipate,bis(3,4-epoxycyclohexylmethyl)pimelate, and the like. Other suitable compounds are described in, for example, U. S. Patent No. 2,750,395.

Other useful cycloaliphatic diepoxides include 2- (3,4-epoxycyclohexyl-5,5-spiro-3,4-epoxy)cyclohexane-m-dioxane, halogen or monovalent hydrocarbon variations of this compound, and the like, as further defined in U.S. Patent No. 3,318,822; cyclopentadiene diepoxide, cyclohexane diepoxide, and the like.

The compositions may include a cycloaliphatic monoepoxide that functions as a reactive diluent and

contributes to overall coating properties. This monoepoxide may be an unsubstituted monoepoxide such as cyclohexene oxide or a substituted monoepoxide with alkyl groups of 1 to 6 carbon atoms, halogen, ester groups, vinyl groups, and the like. Illustrative of the monoepoxides one can mention limonene monoepoxide, 4-vinyl cyclohexene monoepoxide, norbornene monoepoxide, alpha-pinene monoepoxide, cyclohexene monoepoxide, and the like. The cycloaliphatic monoepoxide may be used in the composition in amounts of from 0 to about 40, preferably from 1 to about 30, and most preferably from 1 to about 20 weight percent of the cycloaliphatic epoxide used.

The preferred cycloaliphatic diepoxides are 3,4-epoxycyclohexylmethyl-3,4-epoxycyclohexane carboxylate, bis(3,4-epoxycyclohexylmethyl)adipate, 2-(3,4-epoxycyclohexyl-5,5-spiro-3,4-epoxy)cyclohexane -m-dioxane, or mixtures thereof.

If desired, minor amount of glycidyl epoxides such as the diglycidyl ethers of Bisphenol-A, diglycidyl ethers of brominated Bisphenol-A, cresol-novolac epoxy resins, epoxy phenol novolac resins, diglycidyl ethers of 1,4-butanediol, and the like, can be used in the radiation cure formulations containing the polylactone epoxides of this invention.

Various vinyl ethers can be used in the radiation cure formulations of this invention. Suitable vinyl ethers are illustrated by the dihydropyranyls and di- (dihydropyranyl) compounds as described in U.S. Patent 4,645,781, diethylene glycol vinyl ether, triethylene glycol vinyl ether, and the like, a number of which have been described by J. V. Crivello, J. L. Lee, and D. A. Conion, Proceedings of Radiation Curing IV, Chicago, IL, p 4-28, Sep. 20-23 (1982).

A variety of polyols can be used in the radiation curable formulations containing the polylactone epoxides. Illustrative of these are the mono-, di-, tri-, and higher functionality caprolactone and other lactone polyols; the alkylene oxide polyols including ethylene oxide, propylene oxide, and copolymeric ethylene oxide/propylene oxide polyols, ethylene-oxide capped propylene oxide polyols alkylene oxide derivatives of esterdiols such as the 4-mole ethoxylate of Esterdiol-204; polymer polyols; polyester polyols including hexane diol adipates, butylene adipates, ethylene glycol adipates; poly(tetramethylene oxide) polyols, polymers with pendant hydroxyl groups such as vinyl chloride/vinyl acetate/vinyl alcohol terpolymers, styrene/allyl alcohol copolymers, vinyl acetate/vinyl alcohol copolymers, and the like. The polyols are present in from zero to about 60 weight percent of the formulation, preferably from zero to 40 weight percent of the formulation.

The photoinitiators which may be used in the radiation curable formulations containing the polylactone epoxides of this invention include one or more of a metal fluoroborate and a complex of boron trifluoride as described in U.S. Patent No. 3,379,653; a bis(perfluoroalkyl- sulfonyl)methane metal salt, as described in U. S. Patent No. 3,586,616; an aryl diazonium compound as described in U. S. Patent No. 3,708,296; an aromatic onium salt of Group VIa elements as described in U. S. Patent No. 4,058,400; an aromatic onium salt of Group Va elements as described in U. S. Patent No. 4,069,055; a dicarbonyl chelate of a Group IIIa-Va element as described in U. S. Patent No. 4,068,091; a thiopyrylium salt as described in U. S. Patent No. 4,139,655; a Group VIb element in an $MF_6^-$ anion where M is selected from phosphorous, antimony, and arsenic as described in U.S. Patent No. 4,161,478; an arylsulfonium complex salt as described in U. S. Patent No. 4,231,951; an aromatic iodonium complex salt and an aromatic sulfonium complex salt, as described in U. S. Patent 4,256,828; and bis(4-(diphenylsulfonio)phenyl)sulfide-bis-hexafluorometallic salts such as the phosphate, arsenate, antimonate and the like as described by W. R. Watt and coworkers in J. Polymer Sci.: Polymer Chem. Ed., 22, 1789 (1984). Preferred cationic photoinitiators include the arylsulfonium complex salts, aromatic sulfonium or iodonium salts of halogen containing complex ions, and aromatic onium salts of Group II, V, and VI elements. Some of such salts are commercially available as FX-512 (3M Co.), UVR-6990 and UVR-6974 (Union Carbide Corp.), UVE-1014 and UVE-1016 (General Electric Co.), KI-85 (Degussa), and SP-150 and SP- 170 (Asahi Denka). The photoinitiators are present in the formulations in amounts of from about 0.1 weight percent to about 10 weight percent, preferably from about 0.2 to about 5 weight percent. When photolyzed these photoinitiators fragment into both cationic species and free radicals and thus make possible the prospect of dual cure systems such as might be obtained from combinations of cycloaliphatic epoxides and ethylenically unsaturated molecules.

Various acrylates and methacrylates may be included in the formulations if desired. Illustrative of such acrylates are the caprolactone acrylates, trimethylolpropane triacrylate, alkoxylated Esterdiol-204 diacrylates, diethylene glycol diacrylate, e-ethylhexyl acrylate, neopentyl glycol diacrylate, urethane acrylates, acrylated epoxides or epoxy acrylates, and the like. Acrylates can constitute up to about 35% of the formulation. If desired, one or more additional photoinitiator of the free radical type may be added to the formulation when acrylates are used. Illustrative of such photoinitiators are 2,2-diethoxyacetophenone, acetophenone, alkyl benzoin ethers, 2,2-dimethoxy-2-phenyl acetophenone, and the like. The free radical-generating are used in amounts of about 0.5 to about 5 percent , preferably from about 0.5 to about 3

percent of the formulation.

The radiation curable compositions containing the polylactone epoxides of this invention may include additives such as oils, particularly silicone oils, surfactants such as silicone/alkylene oxide copolymers, acrylic polymers such as those sold under the the MODAFLOW trademark (available from Monsanto Co.), silicone oils containing epoxide groups, fluorocarbon surfactants, low molecular weight alcohols, alkoxy alcohols such as butyl CELLOSOLVE or propoxy ethanol, dialkoxy alcohols such as butyl CARBITOL, and the like, as well as other ingredients known to those skilled in the art of coating formulation. If desired, one may include in the radiation curable compositions various conventional, non-basic fillers (e.g., silica, talc, glass beads or bubbles, microspheres, aluminum trihydrate, clays, etc.) and other additives such as rubbers, tackifying agents, non-basic pigments, and the like, that will not unduly interfere with the photoinitiated polymerization reactions.

Thermally-curable coatings can be made from the above described radiation-curable coatings by deleting the above described photoinitiators from the formulation and substituting a suitable cationic or protonic acid catalyst such as triflic acid salts, boron trifluoride etherate, boron trifluoride, and the like; however, in certain cases of dual radiation-thermal cure system it may be desireable to use both a photoinitiator and a catalyst. Particularly well suited catalysts are the triflic acid salts because of the high quality color (i.e., low color) characteristics of these compounds and the shelf-life characteristics of coatings formulated with many of these compounds. Illustrative of these salts are diethylammonium triflate, which is available from 3M Co. as FC-520, triethylammonium triflate, di-isopropylammonium triflate, ethyl,di-isopropylammonium triflate, and the like, many of which are described by R. R. Alm in the October 1980 issue of Modern Coatings. The amount of catalyst used in the thermally cured formulations is from about 0.05 percent by weight to about 2 percent by weight, preferably from about 0.1 percent by weight to about 1.0 percent by weight, and most preferably from about 0.2 percent by weight to about 0.8 percent by weight of the formulated coating when no pigment or filler is present.

Other thermally cured coatings can be made from the above radiation-curable formulations by deleting the described photoinitiators and conventional cycloaliphatic epoxides from the formulation and substituting a suitable acidic catalyst and adding a crosslinking agent such as an amino resin; however in certain cases of dual radiation-thermal cure systems it may be desireable to use both a photoinitiator and a catalyst and conventional cycloaliphatic epoxides. Suitable amino resins are illustrated by hexamethoxymelamine, butylated melamines, other alkylated melamines, aminoplasts, benzoguanamines, urea formaldehydes, and the like. Suitable catalytsts are illustrated by para-toluene sulfonic acid, methane sulfonic acid, mineral acids, dodecylbenzene sulphonic acid, trimelletic acid, and the like. The catalysts are usually used in amounts of about 0.1 weight percent to about 5 weight percent, preferably from about 0.1 weight percent to about 2 weight percent. Such coatings are cured at temperatures of about 100 °C to over about 250 °C, but preferably from about 120 °C to 200 °C. The amount of amino resin and time of cure at a given temperature depend on the particular formulation and are well known to those skilled in the art of amino-resin chemistry.

In addition to having broad utility in the above described areas, the polylactone epoxides, isocyanate modified polylactone epoxides, and anhydride/carboxylic acid modified polylactone epoxides of this invention are useful as acid scavengers. They may be incorporated into polymeric vinyl chloride inks, coatings, etc., as well as used in other areas that require removal of acids. The hydroxyl-terminated polylactone epoxides have the special effect of being anchorable to substrates through the hydroxyl group, leaving the cycloaliphatic epoxide portion of the molecule available to scavenge acids.

The following examples are illustrative of the present invention and are not intended as a limitation upon the scope thereof. As used in the examples appearing hereinafter, the following designations, terms, and abbreviations have the indicated meanings.

## GLOSSARY

Cycloaliphatic Epoxide-1 is 3,4-epoxycyclohexylmethyl-3,4-epoxycyclohexane carboxylate which is sold by Union Carbide Chemicals and Plastics Company, Inc. (hereinafter Union Carbide) as ERL-4221 and under the tradename CYRACURE® UVR-6110.

Cycolaliphatic Epoxide-2 is bis(3,4-epoxycyclohexyl-methyl)adipate which is sold by Union Carbide as ERL- 4299.

Cycloaliphatic Epoxide-3 is a low viscosity cycloaliphatic epoxide product marketed by Union Carbide as CYRACURE UVR-6100.

Epoxide-4 is the diglycidyl ether of Bisphenol A and is marketed by Shell Chemical Company as EPON® 828.

Polyol-1 is a trifunctional caprolactone polyol having an average molecular weight of 540, an average hydroxyl number of 310, and is available from Union Carbide as TONETM-0305.

Polyol-2 is a difunctional poly(tetramethylene oxide) polyol with an average equivalent weight of 500 and is sold by Quaker Chemical Co. as POLYMEG® 1000.

Polyol-3 is a trifuncitonal propylene oxide polyol having an average equivalent weight of 501, an average hydroxyl number of 112, and is commercially available from Union Carbide.

Photoinitiator-1 is a solution of an aryl sulfonium hexafluoroantimonate salt dissolved in a solvent and is commercially available from General Electric Co. as UVE-1014 or from Union Carbide as CYRACURE UVI-6974.

Photoinitiator-2 is a solution of a aryl sulfonium hexafluorophosphate salt dissolved in a solvent and is commercially available from General Electric Co. as UVE-1016 or Union Carbide as CYRACURE UVI-6990, or in a different solvent from 3M Co. as FX-512.

Catalyst-1 is a solution of diethylammonium triflate and is available from 3M Co. as FC-520.

Surfactant-1 is a silicone/alkylene oxide surfactant that was available from Union Carbide as SILWET® L-5410.

Bonderite 37 steel panels are zinc phosphate-treated steel panels marketed by Parker Co. of the Oximated Industries Corp.

The coating compositions prepared in the examples below were evaluated according to the following procedures.

Solvent Resistance (Double Acetone Rubs): A measure of the resistance of the cured film to attack by acetone, in which a film coating surface is rubbed with an acetone-soaked cloth back and forth with hand pressure. A rub back and forth over the film coating surface with the acetone soaked cheesecloth is designated as one "double acetone rub." The effect that a certain number of double acetone rubs has on the film coating surface is reported by a number in parenthesis following the number of double acetone rubs. The rating system for evaluating acetone resistance for a given number of double acetone rubs is as follows:

Number in Parenthesis After Number of Rubs:

(1) No change in coating appearance
(2) Surface scratched
(3) Surface dulled or marred. some coating removed
(4) Breaks in coating apparent
(5) About 50% or more of coating removed

Pencil Hardness: Pencil leads of increasing hardness values are forced against the film coating surface in a precisely defined manner as described in ASTM D3363-74 until one pencil lead cuts through the surface of the film coating. The surface hardness is considered as the hardest pencil grade which just fails to cut or mar the film coating surface. The pencil leads, in order of softest to hardest, are reported as follows: 6B, 5B, 4B, 3B, 2B, B, HB, F, H, 2H, 3H, 4H, 5H, 6H, 7H, 8H, AND 9H.

Crosshatch Adhesion. A lattice pattern with ten cuts in each direction is made in the coating film to the substrate, and pressure-sensitive adhesive tape (Scotch Brand 606) is applied over the scored/cut substrate and then quickly removed. The amount of coating remaining on the scored area is the "Percent Crosshatch Adhesion."

Gardner Impact Resistance. A measure of the ability of a cured film coating on a substrate to resist rupture from a falling weight. A Model IG-1120 Gardner Impact Tester equipped with an eight-pound dart is used to test film coatings cast and cured on steel panels. The dart is raised to a given height in inches and dropped onto either the coated side of the coated steel panel (direct or forward impact resistance) or the uncoated side of the coated steel panel (reverse impact resistance). The height-of-drop in inches times weight of dart (8 pounds), designated as inch- pounds, absorbed by the film without rupturing is recorded as the film's direct or reverse impact resistance.

EXAMPLES

1. Preparation of an unsaturated polycapro- lactone. To a one-liter, 4-necked, glass reaction vessel equipped with a stirrer, nitrogen sparge inlet and outlet, and thermometer, 222 grams (2.0 mol) tetrahydrobenzyl alcohol (Formula IX) and 513 grams (4.5 moles) of epsilon- caprolactone were added. This mixture was heated to 160 °C and nitrogen sparged at this temperature for four hours. Then, 0.03 gram of stannous octanoate catalyst was added and the reaction was allowed to proceed for 3 hours. At the end of this period, the reaction was allowed to cool to room temperature and remain at this temperature overnight. The reaction mixture was then heated to 195 °C and when at temperature, an additional 0.03 gram of

stannous octanoate catalyst was added and the reaction was allowed to proceed for seven hours at 195° C. After this time, it was cooled to room temperature and stored for future use. A second quantity of the product was prepared in the same way from the same reactants and the two runs were combined. Analysis indicated that the unsaturated polycaprolactone had a viscosity of 128 centistokes at 25° C, an hydroxyl number of 187, and an acid number of 0.11.

Example 2. Preparation of a polycaprolactone epoxide. A reactor system comprising a 3-liter, 3-necked, round-bottomed, glass reaction flask equipped with a mechanical stirrer, thermowatch/thermometer, and y-adapter fitted with an addition funnel and a reflux condenser was mounted over an ice/water bath set on a mechanical jack. Also available was an electric heat gun to aid in temperature regulation. In addition to being available for temperature regulation, the ice/water bath was also available for quenching any large exotherm that might occur. Five hundred and fifty (550) grams of the Example 1 product and 1,000 grams of ethyl acetate were added to the glass reaction flask and this mixture was heated to a reaction temperature of 45° C. When the temperature was controlled at the reaction temperature, 744 grams of a 22.98% by weight solution of peracetic acid in ethyl acetate was carefully added to the reaction flask over a one-hour period. The exothermic reaction was controlled by rate of peracetic acid addition and the temperature regulator. After addition was complete, the reaction mixture was allowed to stir at 45° C for three additional hours and then cooled to 0° C and stored at this temperature until solvent removal was begun. The reaction material was purified by passing it through a Pope evaporator four times using a jacket temperature of 80° C and full laboratory vacuum. The first pass through the evaporator removed most of the ethyl acetate solvent and most of the acetic acid that formed in the epoxidation reaction. The second through fourth passes through the evaporator removed acetic acid and gives the product a low acid number. If desired, the reaction product can be water-washed one or more times to remove acetic acid and any other water-soluble compunds before it is passed through the Pope evaporator. The final product had a yellow color, an acid number of 0.22, and an average epoxide equivalent weight of 298, and a tin content of 9 parts per million. Chromatographic analysis indicated that the polycaprolactone epoxide product had a distribution of molecular weights with some molecules containing up to about 8 molecules of caprolactone in the molecular chain. The average number of caprolactone units per molecule was 1.6 from the average epoxide equivalent weight.

Example 3. Preparation of an isocyanate-modified unsaturated polycaprolactone (Formula IV- and XIV-type products) and an isocyanate modified polycaprolactone epoxide (VI). To a two-liter, four-necked, glass reaction flask equipped with a stirrer, thermometer, nitrogen inlet and outlet, and a side arm for feeding ingredients, 305 grams of Example 1 product, 131 grams of 4,4'-dicyclohexylmethane diisocyanate, and 0.40 gram of dibutyltin dilaurate catalyst were added while stirring. When the catalyst was added, the mixture exothermed to 90° C without an external heat source. The temperature then was increased to 100° C and reaction mixture was held at this temperature for 3 hours to form the isocyanate modified unsaturated polycaprolactone, which was allowed to cool to room temperature and stored for future use.

The isocyanate-modified unsaturated polycaprolactone was cooled to 40° C and 200 grams of ethyl acetate were added. The mixture was then cooled to 5° C in an ice/water bath and 418 grams of 20% percent by weight peracetic acid in ethyl acetate solution were added over a two-hour period. During this addition period the temperature was maintained between 5 °C and 10 °C. After all the peracetic acid solution was added, the reaction mixture was allowed to react overnight while immersed in the ice/water bath. The ethyl acetate solvent and acetic acid formed during the reaction were removed by evaporation in a Roto Film evaporator at 70-80 °C under full laboratory vacuum. The isocyanate modified polycaprolactone epoxide had an oxirane oxygen content of 2.61 weight percent.

Example 4. Preparation of an unsaturated polycaprolactone diol. To a 250 milliliter, 4-necked, glass reaction vessel equipped with a stirrer, nitrogen sparge inlet and outlet, and thermometer, 71 grams (0.5 mol) 3-cyclohexene-1,1-dimethanol (Formula X) and 137 grams (1.2 moles) of epsilon-caprolactone were added. This mixture was heated with stirring to 160° C while sparging with nitrogen. Then, 0.01 gram of stannous octanoate catalyst was added and the temperature was increased to 195° C. The reaction was allowed to proceed at this temperature for 4 hours after which time 0.01 gram of stannous octanoate was added. The mixture was kept at the reaction temperature for an additional 6 hours and cooled to room temperature. The unsaturated polycaprolactone diol was stored for future use.

Example 5. Preparation of a dihydroxyl functional polycaprolactone epoxide. One hundred and thirty grams of the dihyrdoxyl functional, unsaturated polycaprolactone of Example 4 and 50 grams of ethyl acetate were placed in a 1-liter, four-necked glass reaction flask equipped with stirrer, thermometer, nitrogen inlet and outlet, and a side arm for feeding ingredients to the reaction flask. The mixture was then cooled to 5 °C in an ice/water bath and 230 grams of a 20% percent by weight peracetic acid in ethyl acetate solution were carefully and slowly added in a manner such that the temperature of the reacting

mass was held at 5 °C. The addition was made over a time period of about one hour. After all the peracetic acid solution was added, the reaction mixture was allowed to react for three hours while immersed in the ice/water bath. After this time period the reactor was removed from the ice/water bath and allowed to warm to room temperature. The reaction was completed by allowing the reaction mass to stir at room temperature for one hour. The ethyl acetate solvent and acetic acid formed during the reaction were removed by evaporation in a Roto Film evaporator at 70-80 °C under full laboratory vacuum. The polylactone epoxide was stored for future use.

Example 6. Preparation of an unsaturated polycaprolactone. To a one-liter, 4-necked, glass reaction vessel equipped with a stirrer, nitrogen sparge inlet and outlet, and thermometer, 222 grams (2.0 mol) tetrahydrobenzyl alcohol (Formula IX) and 513 grams (4.5 moles) of epsilon-caprolactone were added. The stirring mixture was heated to 160 °C while sparging with nitrogen. Then, 0.03 gram of stannous octanoate catalyst was added, the reaction was allowed to proceed for 3 hours. An additional 0.03 gram of stannous octanoate was added the temperature was increased to 195 °C. and the reaction was allowed to proceed at this temperature for about six hours. The product was cooled to room temperature and stored for future use. The unsaturated polycaprolactone had an hydroxyl number of 140.8 which corresponded to an equivalent weight (number average) and molecular weight of 398.

Example 7. Preparation of an isocyanate modified unsaturated polycaprolactone (Formula IV-and XIV-type products). To a one-liter, four-necked, glass reaction flask equipped with a stirrer, thermometer, and nitrogen inlet and outlet, 300 grams of Example 6 product, 65 grams of toluene diisocyanate, and 0.3 gram of dibutyltin dilaurate catalyst were added while stirring. When the catalyst was added, the mixture exothermed to 90 °C without an external heat source. The temperature was then increased to 100 °C and the reaction mixture was held at this temperature for 3 hours to form the isocyanate modified unsaturated polycaprolactone, which was cooled to room temperature and stored for future use.

Examples 8 and 9. Preparation of an unsaturated polycaprolactones of different average molecular weights. To a one-liter, 4-necked, glass reaction vessel equipped with a stirrer, nitrogen sparge inlet/outlet, condenser and thermometer, the following ingredients were added.

| | Ex. 8 | Ex. 9 |
|---|---|---|
| tetrahydrobenzyl alcohol (Formula IX) | 224 g (2.0 mol) | 224 g (2.0 mol) |
| epsilon-caprolactone | 171 g (1.5 mol) | 285 g (2.5 mol) |

The stirring mixture was heated to 160 °C while sparging with nitrogen. Then, 1 drop (previous determination demonstrated that each drop was equivalent to 0.1 gram of cataltyst) of stannous octanoate catalyst was added and the reaction was allowed to proceed at temperature for 3 hours. The temperature was then increased to 195 °C. and an additional 1 drop of stannous octanoate was added. The reaction was allowed to proceed at this temperature for about 7.5 hours. The product was cooled to room temperature and stored for future use

Example 10. Preparation of an isocyanate modified unsaturated polycaprolactone (Formula IV-and XIV-type products) and an isocyanate modified polycaprolactone epoxide (VI). Three hundred grams of Example 6 product were weighed into a two-liter, four-necked, glass reaction flask equipped with a stirrer, thermometer, nitrogen inlet and outlet, and a side arm for feeding ingredients. While stirring and sparging with nitrogen, the contents were heated to 80 °C. Then 94.7 grams of 4,4'-dicyclohexylmethane diisocyanate were added dropwise while maintaining the temperature at 80 °C. After the last addition of diisocyanate, the reacting mixture was held at 80 °C for one hour. The isocyanate modified unsaturated polycaprolactone was allowed to cool to 35 °C.

With the unsaturated compound at 35 °C, 100 grams of ethyl acetate were added and the mixture was cooled to 15 °C in an ice/water bath. Then 337 grams of a 20 percent by weight solution of peracetic acid in ethyl acetate were added dropwise. The mixture was allowed to stand in the melting ice/water bath overnight (approximately 18 hours). Then, with the reaction mixture at room temperature, 200 milliliters of diethyl ether were added, and then this mixture was poured into a separatory funnel after which 500 milliliters of distilled water were added. The system was agitated, and the liquid portion, consisting of acetic acid that had been formed from the epoxidation reaction, ethyl acetate, diethyl ether and water, was removed by decantation. The product was further washed twice with distilled water to increase the removal

of residual acetic acid. Then 200 milliliters of diethyl ether were added, mixed well with the polycaprolactone epoxide, allowed to separate, and removed by decantation. The product was stripped under laboratory vacuum at 80 °C, cooled to room temperature and stored for future use.

Example 11. Description of the use of an unsaturated polycaprolactone in an ultraviolet light activated, cationically cured coating and an adhesive. The following ingredients were combined in a glass container, stirred, and cast onto Bonderite No. 37 steel panels with a No. 20 wire-wound rod. All quantities are parts by weight. The compositions were cured by exposure to the ultraviolet light radiation from a 100 watt-per-inch, medium-pressure mercury vapor lamp at a conveyor speed of 30 feet per minute.

| | Example | | |
|---|---|---|---|
| | 11 | 12 | 13 |
| **Ingredients** | | | |
| Example 9 Product | 2.5 | 5.0 | 2.5 |
| Cycloaliphatic Epoxide-1 | 7.5 | 5.0 | 7.5 |
| Polyol-1 | --- | --- | 2.1 |
| Photoinitiator-1 | 0.4 | 0.4 | 0.4 |
| Surfactant-1 | 0.05 | 0.05 | 0.05 |
| **Cured Film Properties** | | | |
| Acetone Double Rubs | 100 (1) | 5 (5) | 40 (4) |
| Pencil Hardness | H | * | F |
| Percent Crosshatch Adhesion | 25 | * | 100 |
| **Gardner Impact, in. lbs.** | | | |
| Direct | 5 | * | >320 |
| Reverse | <5 | * | >320 |

*After ultraviolet light exposure and cure this system had a light tack, as is associated with some pressure sensitive adhesives.

The coating of Example 11 is characterized as hard with excellent solvent resistance, of Example 12 as having adhesive characteristics, and of Example 13 as hard with fair solvent resistance and excellent impact resistance.

Examples 14-18. Exemplification of coating formulations containing polycaprolactone epoxides curable with ultraviolet light. The following ingredients are placed in amber-colored glass bottles and stirred well. They are coated onto Bonderite No. 37 steel panels and onto heavy paper stock with a No. 20 wire-wound rod, and exposed to a 300 watt-per-inch, medium pressure mercury vapor light source at 20 feet per minute. Smooth, glossy, attractive coatings result.

| | Example | | | | |
|---|---|---|---|---|---|
| | 14 | 15 | 16 | 17 | 18 |
| Example 2 Product | 15.0 | ---- | 35.0 | 5.0 | 25.0 |
| Example 5 Product | ---- | 15.0 | ---- | ---- | ---- |
| Cycloaliphatic Epoxide-1 | 25.0 | 52.5 | ---- | ---- | ---- |
| Cycloaliphatic Epoxide-2 | 20.0 | ---- | ---- | ---- | ---- |
| Cycloaliphatic Epoxide-3 | 25.5 | 25.0 | 59.5 | 81.5 | 56.0 |
| Epoxide-4 | ---- | ---- | ---- | ---- | 5.0 |
| Polyol-1 | ---- | 5.0 | ---- | ---- | 5.0 |
| Polyol-2 | 10.0 | ---- | ---- | ---- | ---- |
| Polyol-3 | ---- | ---- | ---- | 12.0 | ---- |
| Triethylene glycol Divinylether | ---- | ---- | 3.0 | ---- | 5.0 |
| Photoinitiator-1 | ---- | 2.0 | 2.0 | 1.0 | 1.5 |
| Photoinitiator-2 | 4.0 | ---- | ---- | ---- | 2.0 |
| Surfactant-1 | 0.5 | 0.5 | 0.5 | 0.5 | 0.5 |

Examples 19-24. Exemplification of thermally cured coatings containing polycaprolactone epoxides. The following ingredients are mixed in suitable glass or metal containers, coated onto tin plate and steel panels, and cured in a forced air oven. The coatings of Example 19-21 are baked at 120 °C for 20 minutes, and the coatings of Examples 22 and 23 are baked at 165 °C for 30 minutes. Smooth, glossy, and adherent coatings result.

| | Example | | | | |
|---|---|---|---|---|---|
| | 19 | 20 | 21 | 22 | 23 |
| Example 2 Product | 20.0 | 32.0 | ---- | 15.0 | ---- |
| Example 5 Product | ---- | ---- | 10.0 | ---- | 10.0 |
| Cycloaliphatic Epoxide-1 | ---- | 63.0 | 50.0 | ---- | ---- |
| Cycloaliphatic Epoxide-2 | 5.0 | ---- | 20.0 | ---- | ---- |
| Cycloaliphatic Epoxide-3 | 65.0 | ---- | ---- | ---- | ---- |
| Polyol-1 | 9.0 | ---- | 10.0 | 48.5 | 49.0 |
| Triethylene glycol Divinylether | ---- | 4.0 | 9.0 | ---- | 5.0 |
| Hexahydroxymethyl Melamine | ---- | ---- | ---- | 81.5 | ---- |
| Butylated Melamine | ---- | ---- | ---- | ---- | 56.0 |
| Catalyst-1 | 0.5 | 0.5 | 0.5 | ---- | 2.0 |
| p-Toluene sulfonic acid | ---- | ---- | ---- | 1.0 | 0.5 |
| Surfactant-1 | 0.5 | 0.5 | 0.5 | 0.5 | 0.5 |
| Methyl Amyl Ketone | ---- | 5.0 | ---- | 25.0 | 20.0 |

Example 25. Preparation of tetrahydrobenzyl alcohol-caprolactone adduct having narrow molecular weight distribution and low residual catalyst. In a particularly preferred embodiment of the invention, all glassware to be used is dried by baking in an oven at 120° C for one hour. The caprolactone and the tetrahydrobenzyl alcohol (THB-OH) should be as pure as possible. Both reactants are separately dried to <50 ppm $H_2O$ either by nitrogen sparging or by passing through a column of molecular sieves into dry bottles. A 12 liter 3-necked round-bottom flask is fitted with heating mantle, mechanical stirrer, thermometer, thermowatch, fritted glass $N_2$ sparger tube and oil bubbler-protected outlet. Into the flask are placed 5707 g (50 moles) dry UHP caprolactone and 2804 g (25 moles) day tetrahydrobenzyl alcohol (THB-OH). The $N_2$ sparge is started (flow 25 ml/min) and the stirrer speed is set to give a good dispersion of bubbles. The temperature is then raised to 120° C and held there for 0.5 hr while sparging. At this point, a 50 ml sample is withdrawn with a dry pipette to check the water content by Karl-Fisher titration and to do a gas chromatographic (GC) determination of the reactant ratio. The $H_2O$ content should be <50 ppm. If this is the case, the remainder of the sample may be used to prepare the catalyst solution (catalyst solution is made up by weighing 0.25 g stannous octoate into a dry 25 ml volumetric flask and diluting to the mark with dry reactant mixture). The reactant mixture molar ratio is adjusted as required to 2.00 ± 0.05. If added reactants are not completely dry, additional sparging may be necessary to reduce the water content to <50 ppm. The mole ratio should be rechecked in case more THB-OH is lost during sparging.

When the reaction mixture is sufficiently dry and the reactant molar ratio is correct, there are added 4.2 ml of a 1% solution (see above; 4.2 ml contains 42 mg, or 5 ppm catalyst) of stannous octoate in dry reactant mixture (an aliquot from the reaction mixture itself may be used) by syringe, and the temperature set point is raised to 160° C. At this point an exothermic reaction should begin, resulting in a temperature rise of 1° C/min (at the 12 liter scale of reaction), peaking near 190° C in approximately 1 hour. After an additional hour the temperature should have fallen to 170° C. The heating mantle is turned off and the mixture allowed to cool with stirring overnight. Caprolactone content of the final product should be <1% (typically, nil), and the acid number <.0.25 mg KOH/g. Final yield is 8342 g.

Higher amounts of catalyst, or prolonged (7 hours or more) cooking times at 160° C may be required to reduce the caprolactone concentration to <1% or to achieve a low acid number. If this is the case, a broad

molecular weight distribution will be obtained (the Mw/Mn ratio will be larger than 1.5), and product of relatively high viscosity will be obtained upon subsequent epoxidation.

The final product is analysed for OH Number, water content, unsaturation (by the mercuric acetate method) and molecular weight distribution (determined using SEC). The polydispersity index of the product of this example is found to be significantly lower than that of the previous examples. In addition, there will be less water-soluble product formed during epoxidation, resulting in higher yields. Also, the residual tin content (from the catalyst) will be very low, on the order of 1ppm, thereby minimizing potential interference with subsequent process reactions, e.g., with mixed isocyanates of differing reactivities.

## Claims

**1.** An unsaturated polylactone of the formula
1. An unsaturated polylactone of the formula'

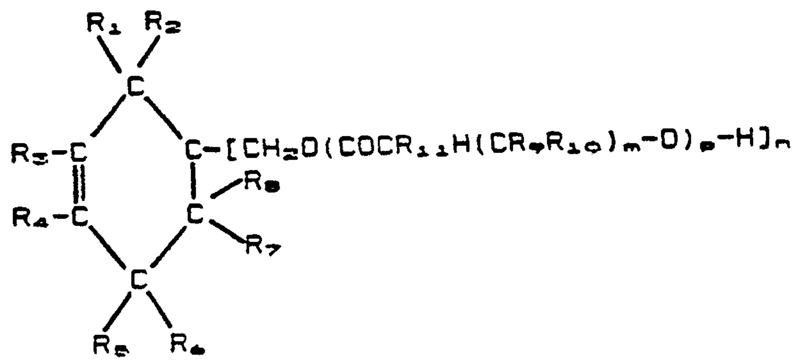

wherein $R_1$ through $R_8$ are the same or different and are hydrogen, phenyl, or unsubstituted or substituted alkyl groups of one to about 8 carbon atoms; n has a value of 1 or 2; $R_9$, $R_{10}$, and $R_{11}$ are the same or different and are hydrogen, phenyl, or lower alkyl having 1 to about 5 carbon atoms with the proviso that not more than 3 of the $R_9$, $R_{10}$, and $R_{11}$ groups are alkyl or phenyl groups; m is an integer of from zero to about 12; and the average value of p is about one to about 30.

**2.** An unsaturated polylactone as in Claim 1 wherein p is from about 1 to about 10.

**3.** An unsaturated polylactone as in Claim 1 wherein $R_1$ to $R_8$ are hydrogen.

**4.** An unsaturated-polylactone as in Claim 3 wherein m is from 3 to 5.

**5.** An unsaturated polylactone as in Claim 4 wherein $R_{11}$ is hydrogen and one $R_9$ or $R_{10}$ is methyl.

**6.** An unsaturated polylactone as in Claim 4 wherein $R_{11}$ is hydrogen and two of $R_9$ and/or $R_{10}$ are methyl.

**7.** An unsaturated polylactone as in Claim 4 wherein $R_9$ to $R_{11}$ are hydrogen.

**8.** An unsaturated polylactone as in Claim 7 wherein m is four.

**9.** A epoxidized derivative of the unsaturated polylactone of Claim 1 with the following formula:

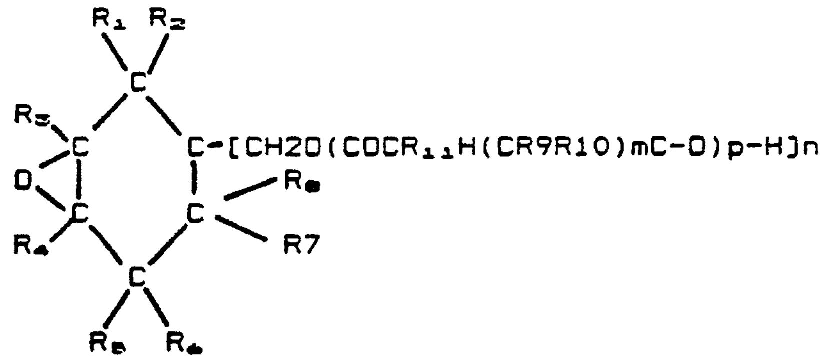

10. A polylactone epoxide as in Claim 9 wherein p is from about one to about ten.

11. A polylactone epoxide as in Claim 9 wherein $R_1$ to $R_8$ are hydrogen.

12. A polylactone epoxide as in Claim 11 wherein m is from 3 to 5.

13. A polylactone epoxide as in Claim 12 wherein $R_{11}$ is hydrogen and one $R_9$ or $R_{10}$ is methyl.

14. A polylactone epoxide as in Claim 12 wherein $R_{11}$ is hydrogen and two of $R_9$ and/or $R_{10}$ are methyl.

15. A polylactone epoxide as in Claim 12 wherein $R_9$ to $R_{11}$ are hydrogen.

16. An unsaturated polylactone as in Claim 15 wherein m is four.

17. An isocyanate-modified derivative of the unsaturated polylactone of Claim 1 with the following formula:

$$(F)_w(-OC\overset{H}{N})_x-(B)_z$$

wherein x has a value of 1 to 6, w has a value of 1 to 3, F has the following structural formula and is the compound of Claim 1 without the active hydrogen atoms:

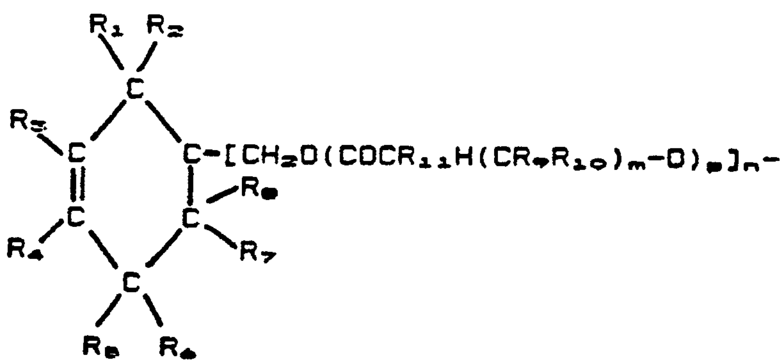

wherein B is the residue of a polyfunctional or monofunctional isocyanate when its isocyanate groups are removed, and z has as a value of 1 or 2.

18. An isocyanate-modified derivative of the unsaturated polylactone of Claim 17 with x having a value of from 1 to 3.

**19.** An isocyanate-modified unsaturated polylactone of Claim 18 with z having a value of 1.

**20.** An isocyanate-modified unsaturated polylactone as in Claim 17 wherein p is from about 1 to 10.

**21.** An isocyanate-modified unsaturated polylactone as in Claim 17 wherein $R_1$ to $R_8$ are hydrogen.

**22.** An isocyanate-modified unsaturated polylactone as in Claim 21 wherein m is from 3 to 5.

**23.** An isocyanate-modified unsaturated polylactone as in Claim 22 wherein $R_{11}$ is hydrogen and one $R_9$ or $R_{10}$ is methyl.

**24.** An isocyanate-modified unsaturated polylactone as in Claim 22 wherein $R_{11}$ is hydrogen and two of $R_9$ and/or $R_{10}$ are methyl.

**25.** An isocyanate-modified unsaturated polylactone as in Claim 22 wherein $R_9$ to $R_{11}$ are hydrogen.

**26.** An isocyanate-modified unsaturated polylactone as in Claim 25 wherein m is 4.

**27.** An isocyanate-modified unsaturated polylactone as in Claim 26 wherein B is the residue from 4,4'-dicyclohexylmethane diisocyanate.

**28.** An isocyanate-modified unsaturated polylactone as in Claim 26 wherein B is the residue from toluene diisocyanate.

**29.** An isocyanate-modified unsaturated polylactone as in Claim 22 where x and w are 1 or 2, z is 1, B is a linear or branched alkyl of 1 to 10 carbon atoms, phenyl, napthyl, or benzene, 1-(1-isocyanato-1-methylethyl)-3-(1-methylethenyl), and has the following structure:

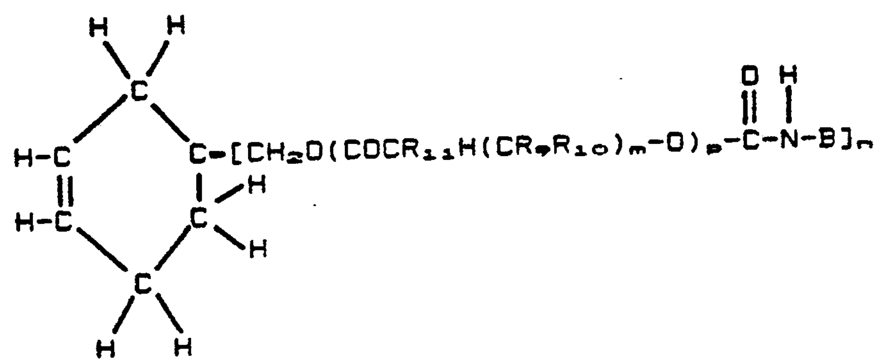

**30.** An anhydride and/or carboxylic acid modified derivative of the unsaturated polylactone of Claim 1 with the following formula:

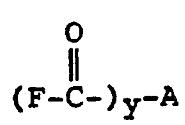

wherein A is the residue of a polyfunctional carboxylic acid without its carboxyl groups or of an anhydride without the anhydride group (-CO-O-CO-), y has a value of 1 to 3, F has the following structural formula and is the compound of Claim 1 without the active hydrogen atoms:

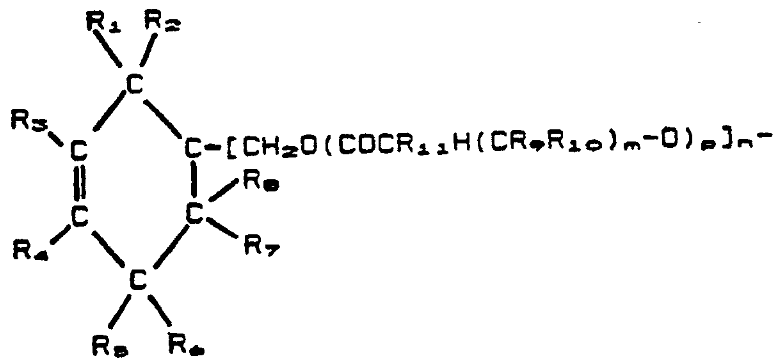

31. An anhydride and/or carboxylic acid modified unsaturated polylactone as in Claim 30 wherein p is from about 1 to about 10.

32. An anhydride and/or carboxylic acid modified unsaturated polylactone as in Claim 30 wherein $R_1$ to $R_8$ are hydrogen.

33. An anhydride and/or carboxylic acid modified unsaturated polylactone as in Claim 32 wherein m is from 3 to 5.

34. An anhydride and/or carboxylic acid modified unsaturated polylactone as in Claim 33 wherein $R_{11}$ is hydrogen and one $R_9$ or $R_{10}$ is methyl.

35. An anhydride and/or carboxylic acid modified unsaturated polylactone as in Claim 33 wherein $R_{11}$ is hydrogen and two of R9 and/or $R_{10}$ are methyl.

36. An anhydride and/or carboxylic acid modified unsaturated polylactone as in Claim 33 wherein $R_9$ to $R_{11}$ are hydrogen.

37. An anhydride and/or carboxylic acid modified unsaturated polylactone as in Claim 36 wherein m is 4.

38. An anhydride and/or carboxylic acid modified unsaturated polylactone as in Claim 33 wherein y is 1, A is a linear or branched alkyl of 1 to 10 carbon atoms, phenyl and has the following structure:

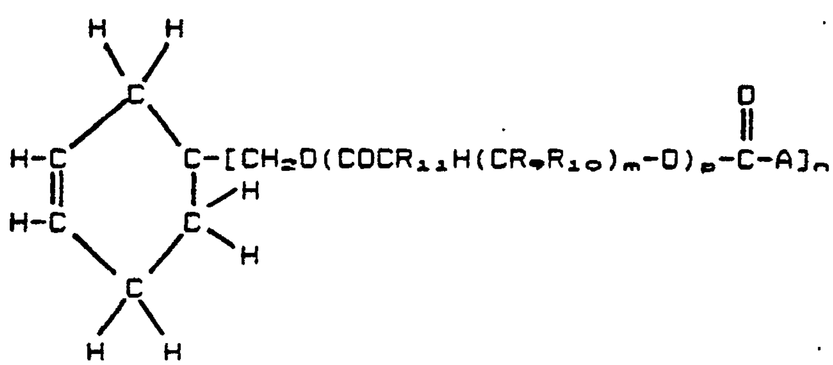

39. An anhydride or carboxylic acid modified unsaturated polylactone as in Claim 38 wherein A is methyl.

40. An epoxidized derivative of the isocyanate- modified derivative of Claim 17 with the following formula

$(E)_w(-OCN)_x-(B)z$ wherein E is an epoxidized form of F and has the following structural formula and is the compound of Claim 9 without the active hydrogen atoms:

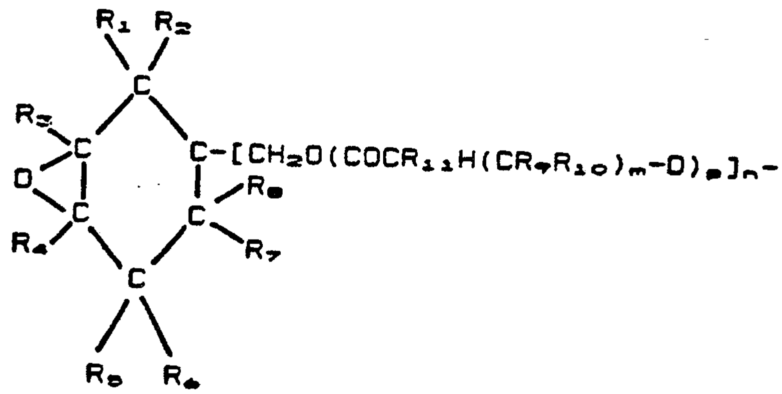

VIII

41. An epoxidized derivative of the anhydride or carboxylic acid modified derivative of Claim 30 with the following formula:

VII

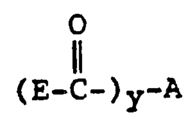

wherein E is an epoxidized form of F and has the following structural formula and is the compound of Claim 9 without the active hydrogen atoms:

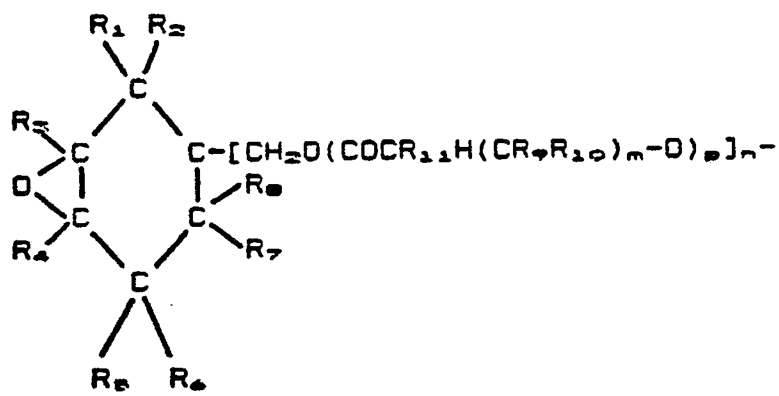

VIII

42. A radiation-curable composition comprising:
   (a) 1 to 60 weight percent of an unsaturated polylactone of Claim 1, 17, or 30 or a mixture thereof,
   (b) 0 to 95 weight percent of cycloaliphatic epoxides,
   (c) 0 to 60 weight percent of a polyol, and
   (d) 0.1 to 10 weight percent of an onium salt photoinitiator.

43. A composition as defined in Claim 42 wherein the unsaturated polylactone is of the following formula:

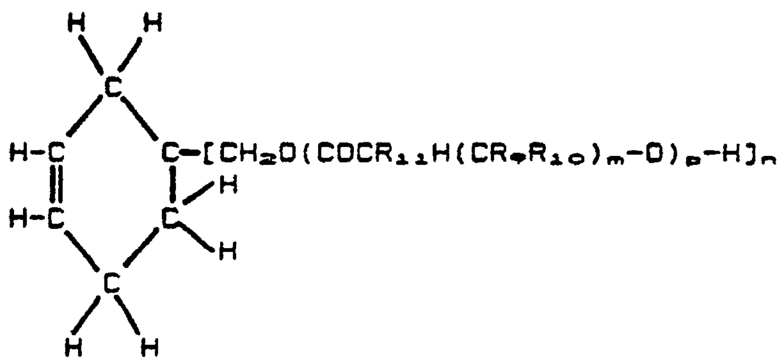

wherein n has a value of one or two; $R_9$, $R_{10}$, and $R_{11}$ are the same or different and are hydrogen, phenyl, or lower alkyl having 1 to about 5 carbon atoms with the proviso that not more than three of the $R_9$, $R_{10}$, and $R_{11}$ groups are alkyl or phenyl groups; m is an integer of from zero to about 12; and the average value of p is about 1 to about 30.

**44.** A composition as in Claim 43 wherein the cycloaliphatic epoxide is 3,4-epoxycyclohexylmethyl-3,4-epoxycyclohexane carboxylate.

**45.** A composition as in Claim 43 wherein the cycloaliphatic epoxide is bis(3,4-epoxycyclohexylmethyl)-adipate.

**46.** A composition as in Claim 44 wherein the polyol is a polycaprolactone polyol.

**47.** A composition as in Claim 46 wherein the photoinitiator is an aryl sulfonium salt.

**48.** A composition as in Claim 46 wherein the photoinitiator is an aryl sulfonium hexafluoroantimonate.

**49.** A radiation-curable composition comprising:

(a) 1 to about 100 weight percent of an polylactone epoxide of Claims 9, 40, or 41 or a mixture thereof,

(b) 0 to 95 weight percent of cycloaliphatic epoxides,

(c) 0 to 60 weight percent of a polyol, and

(d) 0.1 to 10 weight percent of an onium salt photoinitiator.

**50.** A composition as defined in Claim 49 wherein the polylactone epoxide is of the following formula:

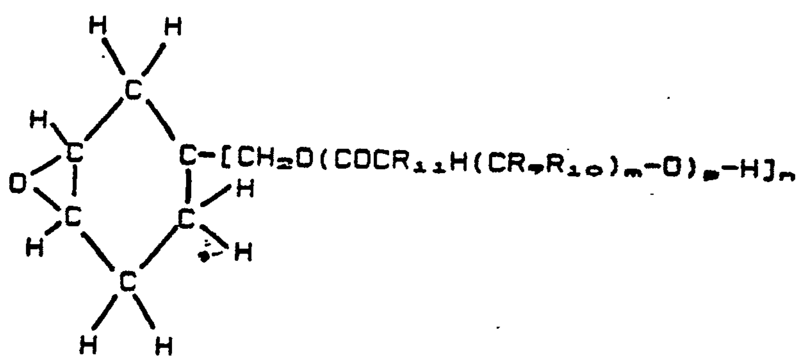

wherein n has a value of one or two; $R_9$, $R_{10}$, and $R_{11}$ are the same or different and are hydrogen, phenyl, or lower alkyl having 1 to about 5 carbon atoms with the proviso that not more than three of the $R_9$, $R_{10}$, and $R_{11}$ groups are alkyl or phenyl groups; m is an integer of from zero to about 12; and the average value of p is about 1 to about 30.

**51.** A composition as in Claim 50 wherein the cycloaliphatic epoxide is 3,4-epoxycyclohexylmethyl-3,4-epoxycyclohexane carboxylate.

**52.** A composition as in Claim 50 wherein the cycloaliphatic epoxide is bis(3,4-epoxycyclohexylmethyl)-adipate.

**53.** A composition as in Claim 51 wherein the polyol is a polycaprolactone polyol.

**54.** A composition as in Claim 53 wherein the photoinitiator is an aryl sulfonium salt.

**55.** A composition as in Claim 53 wherein the photoinitiator is an aryl sulfonium hexafluoroantimonate.

**56.** A thermally-curable composition comprising:
(a) 1 to 60 weight percent of an unsaturated polylactone of Claims 1, 17, or 30 or a mixture thereof,
(b) 0 to 95 weight percent of cycloaliphatic epoxides,
(c) 0 to 60 weight percent of a polyol, and
(d) 0.05 to 2 weight percent of a thermally activated cationic catalyst.

**57.** A composition as defined in Claim 56 wherein the unsaturated polylactone is of the following formula:

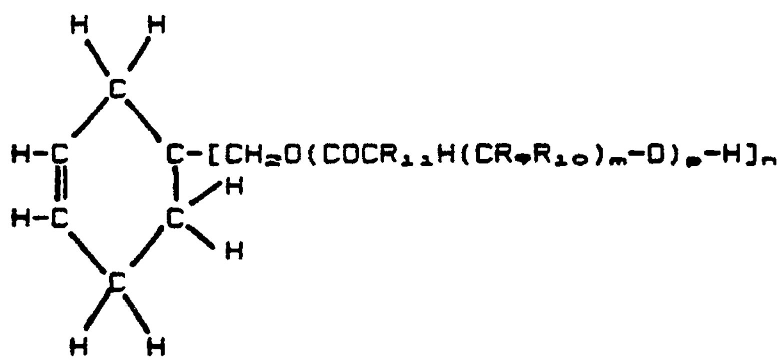

wherein n has a value of one or two; $R_9$, $R_{10}$, and $R_{11}$ are the same or different and are hydrogen, phenyl, or lower alkyl having 1 to about 5 carbon atoms with the proviso that not more than three of the $R_9$, $R_{10}$, and $R_{11}$ groups are alkyl or phenyl groups; m is an integer of from zero to about 12; and the average value of p is about 1 to about 30.

**58.** A composition as in Claim 57 wherein the cycloaliphatic epoxide is 3,4-epoxycyclohexylmethyl-3,4-epoxycyclohexane carboxylate.

**59.** A composition as in Claim 57 wherein the cycloaliphatic epoxide is bis(3,4-epoxycyclohexylmethyl)-adipate.

**60.** A composition as in Claim 58 wherein the polyol is a polycaprolactone polyol.

**61.** A composition as in Claim 60 wherein the catalyst is a triflic acid salt.

**62.** A composition as in Claim 61 wherein the triflic acid salt catalyst is a solution of diethylammonium triflate.

**63.** A thermally-curable composition comprising:
(a) 1 to about 100 weight percent of an polylactone epoxide of Claims 9, 40, or 41 or a mixture thereof,
(b) 0 to 95 weight percent of cycloaliphatic epoxides,
(c) 0 to 60 weight percent of a polyol, and
(d) 0.05 to 2 weight percent of a thermally activated cationic catalyst.

**64.** A composition as defined in Claim 63 wherein the polylactone epoxide is of the following formula:

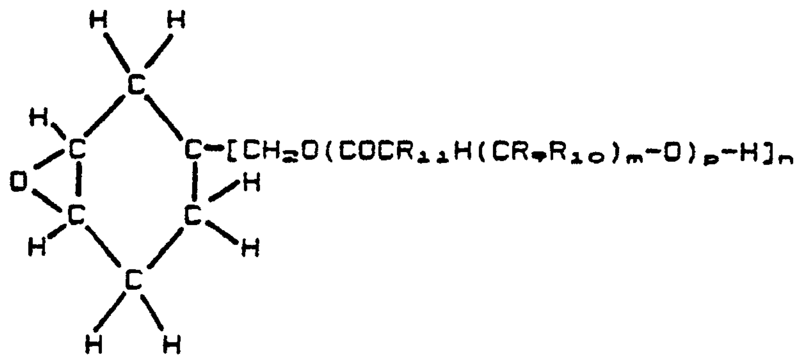

wherein n has a value of one or two; $R_9$, $R_{10}$, and $R_{11}$ are the same or different and are hydrogen, phenyl, or lower alkyl having 1 to about 5 carbon atoms with the proviso that not more than three of the $R_9$, $R_{10}$, and $R_{11}$ groups are alkyl or phenyl groups; m is an integer of from zero to about 12; and the average value of p is about 1 to about 30.

**65.** A composition as in Claim 64 wherein the cycloaliphatic epoxide is 3,4-epoxycyclohexylmethyl-3,4-epoxycyclohexane carboxylate.

**66.** A composition as in Claim 64 wherein the cycloaliphatic epoxide is bis(3,4-epoxycyclohexylmethyl)-adipate.

**67.** A composition as in Claim 65 wherein the polyol is a polycaprolactone polyol.

**68.** A composition as in Claim 67 wherein the catalyst is a triflic acid salt.

**69.** A composition as in Claim 68 wherein the triflic acid salt catalyst is a solution of diethylammonium triflate.

**70.** A thermally-curable composition comprising:
(a) 1 to 60 weight percent of an unsaturated polylactone of Claims 1, 17, or 30 or a mixture thereof,
(b) 0 to 95 weight percent of an amino resin,
(c) 0 to 60 weight percent of a polyol, and
(d) 0.05 to 2 weight percent of an acid catalyst.

**71.** A thermally-curable coating composition as in Claim 70 wherein the acid catalyst is para-toluene sulfonic acid.

**72.** A thermally-curable composition comprising:
(a) 1 to about 100 weight percent of an polylactone epoxide of Claims 9, 40, or 41 or a mixture thereof,
(b) 0 to 95 weight percent of an amino resin,
(c) 0 to 60 weight percent of a polyol, and
(d) 0.05 to 2 weight percent of an acid catalyst.

**73.** A thermally-curable coating composition as in Claim 72 wherein the acid catalyst is para-toluene sulfonic acid.

**74.** A cured polymeric film or article from the composition of Claim 72.

**75.** A process for preparing polylactone epoxide compositions which comprises:
(a) reacting an unsaturated alcohol with a lactone in the presence of less than about 500 parts per million catalyst at a temperature of from about 100 °C to about 200 °C,
(b) cooling said unsaturated polylactone to a temperature of from about 5 °C to about 90 °C,
(c) slowly adding a solution of a peracid epoxidizing agent and allowing reaction to proceed at a

temperature of from about 5 ° C to about 90 ° C to form the polylactone epoxide,
(d) optionally washing with water and decanting to recover the organic layer,
(e) vacuum stripping the solvent, acid formed, and remaining reactants from the polylactone epoxide.

**76.** A process as defined in Claim 75 wherein the lactone is epsilon-caprolactone.

**77.** A process as defined in Claim 75 wherein the catalyst is stannous octanoate.

**78.** A process as defined in Claim 75 wherein the catalyst concentration is less than about 50 parts per million.

**79.** A process for preparing isocyanate-modified polylactone epoxide compositions which comprises:
(a) reacting an unsaturated alcohol with a lactone in the presence of less than about 500 parts per million catalyst at a temperature of from about 100 ° C to about 200 ° C for from about 2 to 24 hours,
(b) cooling said unsaturated polylactone to a temperature of from about 25 ° C to about 100 ° C and slowly adding a monofunctional isocyanate, polyfunctional isocyanate, or mixure of isocyanates to the unsaturated polylactone and reacting at a temperature of from about 25 ° C to about 100 ° C for a period of time of from about 1 to about 8 hours,
(c) changing the temperature to from about 5 ° C to about 90 ° C, and slowly adding a solution of a peracid epoxidizing agent to the isocyanate-modified unsaturated polylactone and allowing the reaction to proceed at a temperature of from about 5 ° C to about 90 ° C to form the isocyanate-modified polylactone epoxide,
(d) optionally washing with water and decanting to recover the organic layer.
(e) vacuum stripping the solvent, acid formed, and remaining reactants from the isocyanate-modified polylactone epoxide.

**80.** A process as defined in Claim 79 wherein the lactone is epsilon-caprolactone.

**81.** A process as defined in Claim 79 wherein the catalyst is stannous octanoate.

**82.** A process as defined in Claim 79 wherein the catalyst is dibutyltin dilaurate.

**83.** A process for preparing anhydride or carboxylic acid modified polylactone epoxide compositions which comprises:
(a) reacting an unsaturated alcohol with a lactone in the presence of less than about 500 parts per million catalyst at a temperature of from about 100 ° C to about 200 ° C for from about 2 to 24 hours,
(b) adjusting the temperature of the unsaturated polylactone formed to a temperature of from about 60 ° C to about 200 ° C and slowly adding an anhydride, a mono-or multifunctional carboxylic acid, or a mixture of anhydrides and carboxylic acids to the unsaturated polylactone and reacting at a temperature of from about 60 ° C to about 200 ° C for a period of time of from about 1 to about 8 hours while removing water formed in the condensation reaction,
(c) changing the temperature to from about 5 ° C to about 90 ° C, and slowly adding a solution of a peracid epoxidizing agent to the anhydride or carboxylic acid modified unsaturated polylactone and allowing the reaction to proceed at a temperature of from about 5 ° C to about 90 ° C to form the anhydride or carboxylic acid modified modified polylactone epoxide,
(d) optionally washing with water and decanting to recover the organic layer,
(e) vacuum stripping the solvent, acid formed, and remaining reactants from the anhydride or carboxylic acid modified polylactone epoxide.

**84.** A process as defined in Claim 83 wherein the lactone is epsilon-caprolactone.

**85.** A process as defined in Claim 83 wherein the catalyst is stannous octanoate.

**86.** A process as defined in Claim 83 wherein the catalyst concentration is less than about 50 parts per million.